# EUROPEAN PATENT APPLICATION

(11) **EP 4 837 496 A1**
(43) Date of publication of application: **11.11.2026**
(21) Application number: 25175450.3
(22) Date of filing: 09.05.2025
(51) Int. Cl.: A61B 1/00, A61B 1/005, A61B 1/01

(54) **ANGULATION ASSEMBLY FOR USE IN AN ENDOSCOPE**

(71) Applicant: PENTAX Medical Corporation, Akishima-shi, Tokyo 196-0012 (JP)
(72) Inventor: WALBERG, Erik, Tokyo, 160-8347 (JP); RIZZO, Marco, Tokyo, 160-8347 (JP); BROST, Eckhardt, Tokyo, 160-8347 (JP)
(74) Representative: Lucke, Andreas

(57) **Abstract**

An angulation assembly for an endoscope, the angulation assembly comprising a body to be held by a human operator and to be coupled to an insertion tube of the endoscope, an angulation structure rotatably mounted inside the body and configured for supporting a first control wire and a second control wire extending from opposite sides of the angulation structure through the body to the insertion tube, such that rotation of the angulation structure winds one of the first and second control wire and unwinds the other one of the first and second control wire, and a tensioning mechanism mounted in the body in a selected tensioning state and coupled to the first and second control wire, wherein a motion of the tensioning mechanism adjusts the tensioning state of the first control wire and the second control wire, when coupled to the insertion tube.

## Description

### FIELD OF THE DISCLOSURE

The present disclosure is in the field of medical technology. In particular, the present disclosure relates to an angulation assembly for use in an endoscope that includes a an actively angulated tube section.

### BACKGROUND

An endoscope is a surgical device that may be used to access (e.g., view or remove) or treat tissue within the body of a patient by inserting one or more medical tools into the body through an incision in the body or an orifice of the body. The endoscope may include (comprise) an interface/control portion and an insertion tube that is coupled to the interface/control portion. The insertion tube is configured to be inserted into the body of the patient and may include one or more channels to provide access to tissue within the body. The one or more channels may e.g. be configured to receive a medical tool and/or a fluid and to guide the medical tool and fluid, respectively, to the tissue of interest.

The insertion tube may be bendable to facilitate insertion into the patient's body. For this, the insertion tube often includes one or more passively and/or actively bendable sections (also referred to as passive and active bending section, respectively). A passively bendable section may e.g. be formed of a bendable or flexible material or structure that can be deformed by applying an external force. An actively bendable section may include a plurality of joints that can be articulated, e.g. for actively navigating a tip of the endoscope at a distal end of the insertion tube. Each of the joints may for example include a circular joint head arranged in a circular joint socket such that the joint head can pivot with respect to the joint socket, thereby allowing for bending the insertion tube.

Known systems typically involve the use of angulation assemblies that allow the operator to control the direction and orientation of the insertion tube. These assemblies often rely on a series of control wires that are coupled to the active bending section and can be manipulated to achieve the desired movement.

### SUMMARY OF THE DISCLOSURE

One of the primary issues with existing angulation assemblies is the difficulty in providing consistent tension in the control wires. Inadequate tension can lead to imprecise movements and reduced control, which can compromise the accuracy of the procedure. Conversely, excessive tension can cause undue stress on the wires, leading to premature wear and potential failure. Known systems often lack a reliable method for adjusting the optimal tension in the control wires, generally relying on gluing of wires and/or of wire sheaths in place during assembly.

Additionally, the complexity of the angulation assemblies in current endoscopes can not only increase the manufacturing cost but also makes it difficult to perform necessary adjustments during use or assembly. Furthermore, angulation assemblies for adjusting the angulation state of the insertion tube are often bulky and cumbersome, limiting the maneuverability of the endoscope and potentially causing discomfort to the patient.

It is thus a feature of the present disclosure to provide an angulation assembly for an endoscope that at least partially overcomes the disadvantages of known systems and can in particular provide a simple but effective system for controlling the endoscope even for single use endoscope designs in which both material selection and complexity may be limited.

According to a first aspect, an angulation assembly for an endoscope is provided. The angulation assembly comprises a body adapted to be held by a human operator. The body is adapted to be coupled to an insertion tube of the endoscope. An angulation structure is rotatably mounted inside the body. The angulation structure is configured for supporting a first control wire and a second control wire. The first control wire and the second control wire extend from opposite sides of the angulation structure through the body to the insertion tube. Rotation of the angulation structure winds one of the first control wire and the second control wire and unwinds the other one of the first control wire and the second control wire. A tensioning mechanism is mounted in the body in a selected tensioning state. The tensioning mechanism is coupled to the first control wire and the second control wire. A motion of the tensioning mechanism adjusts the tensioning state to increase or reduce a tension of the first control wire and the second control wire, when coupled to the insertion tube.

The body may be designed to be ergonomically held by a human operator, ensuring ease of use and precise control during endoscopic procedures. The angulation structure is rotatably mounted inside the body allowing the angulation structure to pivot or rotate, providing the necessary movement to control the control wires. The angulation structure is configured for supporting a first control wire and a second control wire, e.g. on a radial outer surface of the angulation structure, such as to transmit the mechanical force required to bend or angle the insertion tube. The angulation structure may comprise an angulation wheel rotatably mounted in the body. The angulation structure may further comprise a guiding structure for holding the control wires on a perimeter of the angulation structure.

For example, the control wires may be prevented from slipping from the angulation structure along an axial direction (along the rotation axis of the angulation structure) through a recessed channel and/or radially protruding webs on a radial surface of the angulation structure for guiding the first control wire and the second control wire along the radial surface of the angulation structure.

The first control wire and the second control wire can be supported on the angulation structure and extend from opposite sides of the angulation structure through the body towards the insertion tube, for ensuring that the angulation assembly can effectively control the movement of the insertion tube in opposite directions. The control wire may for example be configured to, when actuated, apply a compressive tension to the insertion tube or a part thereof, in particular an active bending section including a plurality of hinged tube elements, along the longitudinal direction, e.g. from the distal end to the proximal end. The control wires may for example be connected or attached to a distal attachment point of the insertion tube, which may for example be arranged on a distal side of the tube elements, e.g. at or adjacent to the distal end or tip of the insertion tube. When pulling on a proximal end of the control wire, a force may be generated from the distal attachment point towards the proximal end of the insertion tube. In some examples, the control wire may also be configured to apply a compressive tension even when not actuated, for example to bias the insertion tube towards the default configuration and/or to secure joint heads of the tube elements in corresponding joint sockets. The control wires may be guided at multiple guiding elements distributed along the extension direction of an active bending section of the insertion tube to facilitate a force transfer from the control wires onto the insertion tube. The angulation assembly may be compatible with different control wire diameters such as between about 0.2 mm and about 0.5 mm. The control wires may be braided or solid wires and may be made of a metal, such as braided steel wires.

The insertion tube may be mounted to the body at a coupling portion of the body, the coupling portion of the body comprising an opening facing the angulation structure, wherein the first and second control wires may extend from the angulation structure towards the coupling portion of the body and through the opening into a channel of the insertion tube. The body may comprise deflection posts, arranged perpendicular to the extension direction of the control wires for deflecting the control wires along a path from the angulation structure towards the opening. The control wires may each be connected to a distal end of the insertion tube, such that modifying an effective length of the first and second control wire pulls along a respective side of the insertion tube for actively angulating the insertion tube.

The first and second control wires may be different sections of the same control wire, which may extend around and from different sides of the angulation structure. Rotation of the angulation structure winds one of the first control wire and the second control wire and unwinds the other one of the first control wire and the second control wire, which may effectively shorten one of the control wire sections, while lengthening the other, such as to controllably angulate the insertion tube.

In some examples, the angulation assembly further comprises a handle accessible from the outside of the body and displacement of the handle induces a rotation of the angulation structure.

The handle can provide user interface for controlling the angulation assembly. The displacement of the handle communicates with the angulation structure, causing it to rotate. The handle may be rotated to drive a rotation of the angulation structure inside of the body. The handle may comprise an axial bracket portion extending between opposite sides of the body, such as to engage the angulation structure on opposite sides along the rotation axis of the angulation structure, which may increase a mechanical stability of the angulation assembly.

In some examples, the handle comprises a protrusion protruding into the body towards the angulation structure, wherein the protrusion protrudes along a rotation axis of the angulation structure.

The protrusion may act as a physical intermediary, transferring the motion from the handle to the angulation structure. The alignment of the protrusion with the rotation axis of the angulation structure may ensure that the force applied via the handle is efficiently converted into rotational motion of the angulation structure. This feature minimizes mechanical losses and enhances the precision of the angulation control. In some examples, the angulation structure is rotatably mounted in the body on the protrusion, and/or the protrusion may be coaxial with and connected to an axial shaft of the angulation structure. The protrusion may constitute a shaft element of the angulation assembly.

In some examples, the angulation structure and the handle are in form-fitting engagement. For example, an axial protrusion of the handle, which can be aligned coaxial with the rotation axis, may comprise a patterned peripheral outer surface and the angulation structure may comprise a corresponding slot having a matching patterned peripheral inner surface, or vice versa, and the patterned peripheral outer surface may be inserted into the corresponding slot having the matching patterned peripheral inner surface. For example, the patterned peripheral outer surface may comprise a radial protrusion protruding radially with respect to the rotation axis, and the slot may comprise a notch aligning with the radial protrusion for a form-fitting engagement, which can enable a torque transfer from the handle to the angulation structure.

The tensioning mechanism may maintain appropriate tension in the control wires, ensuring that they remain taut and responsive to the operator's inputs. The tensioning mechanism is coupled to the first control wire and the second control wire. In some examples, the tensioning mechanism also functions as a mounting structure for mounting the control wires on the angulation structure. Motion of the tensioning mechanism adjusts the tensioning state to increase a tension of the first control wire and the second control wire, when coupled to the insertion tube, along a first movement direction, and to decrease the tension of both control wires when moved along second opposite movement direction. The motion of the tensioning structure may be a movement of a single component of the tensioning structure, for adjusting the tension in both wires with a single component, e.g. by turning or sliding the tensioning component, which should preferably by directly coupled, e.g. in physical contact, with both control wires. This adjustability allows to fine-tune the tension in the wires, e.g. during assembly, optimizing the responsiveness and control of the angulation assembly. Preferably, the motion of the tensioning mechanism symmetrically increases (decreases) the tension of the first control wire and the second control wire, e.g. by symmetrically shortening (lengthening) an effective length of both control wires in the active bending section of an insertion tube coupled to the body.

In some examples, the tensioning mechanism comprises a spindle mounted on the angulation structure and coupled to the first control wire and the second control wire, wherein rotation of the spindle in a (first) tensioning direction winds up both the first control wire and the second control wire onto the spindle.

The spindle's coupling to both the first and second control wires allows it to simultaneously affect the tension in both wires, ensuring synchronized adjustments. This feature brings the advantage of a unified tensioning system, which can simplify the operation and maintenance of the angulation assembly. Conversely, turning the spindle in a (second) unwinding direction can symmetrically unwind both the first control wire and the second control wire to reduce the tension in the control wires. The spindle may act as an integrated mounting and tensioning mechanism for the control wires during assembly of the angulation assembly.

In some examples, the spindle is a screw type fastener, wherein the screw type fastener is screwed into the angulation structure for increasing the tension of the first control wire and the second control wire.

The control wires may be coupled to the screw type fastener during assembly, and screwing the screw type fastener into the angulation structure may at the same time mount the first control wire and the second control wire on the angulation structure and increase the tension of the first control wire and the second control wire.

The screw type fastener may employ a threaded mechanism to achieve the winding and tensioning of the wires during the mounting operation of the screw type fastener. Thus, the screw type fastener may be screwed into the angulation structure for increasing the tension of the first control wire and the second control wire.

The integration of the screw type fastener as a tensioning mechanism and mounting structure for the control wires may ensure that the tensioning mechanism is more compact.

In some examples, the first control wire and the second control wire are threaded through a recess or aperture of the spindle.

The first control wire and the second control wire may be portions of a common control wire, which may be threaded through a through-hole or notch located on the spindle, with the spindle delimiting the first control wire and the second control wire. In some examples, the first control wire and the second control wire are joined to each other, e.g. at the spindle. This joining could be achieved through various means such as welding, soldering, or using a mechanical fastener like a clamp or a crimp. The recess or aperture in the spindle can safely hold the wires for a reliable mounting of the control wires, in particular by facilitating the assembly of the angulation assembly in a neutral state, in which the control wire tension is equal in the first and second control wires, which configuration may be fixed through a winding of the control wires onto the spindle. The recess or aperture may for example be arranged on a top portion of a screw type fastener.

Alternatively or additionally, the control wires may be wound around the spindle, and optionally fastened at the spindle with a knot, prior to winding up the first control wire and the second control wire on the spindle, e.g. by screwing a screw type fastener acting as the spindle into the angulation structure.

The control wires may be wires of cable pulls, which may be sleeved in a wire sleeve at least through a portion of the insertion tube, e.g. through a passive bending section of the insertion tube, such as to prevent active angulation of the respective portion of the insertion tube and to limit the active angulation to an active bending section of the insertion tube, e.g. close to a distal end of the insertion tube, while the passive bending section may be free to bend, e.g. to accommodate a (winding) path through a biological lumen. The wire sleeve may be fixedly connected to an element mounted in the body at a first end of the wire sleeve and to a distal end of a passive bending section of the insertion tube at an opposite (second) end of the wire sleeve.

In some examples, the tensioning mechanism comprises a slider comprising mounting points for fixedly holding ends of a first sheath and a second sheath sheathing the first control wire and the second control wire, respectively.

The first sheath and the second sheath may be respective wire sleeves for the first and second control wires for implementing corresponding Bowden-type cable pulls. The first and second sheaths can encase the first control wire and the second control wire, respectively. When the first and second control wires are mounted on the angulation structure with a respective first end, and mounted to the insertion tube with a second end, the slider may interact with the sheaths and the control wires, ensuring that the wires are properly tensioned.

The motion of the tensioning structure may comprise a movement of the slider, for adjusting the tension in both wires with a single component, e.g. by sliding of the slider, which may be directly coupled, e.g. in physical contact with the first and second control wire sheaths.

In some example, the slider may be configured for linear translation with respect to the angulation structure to adjust a distance between the angulation structure and the ends of the first sheath and the second sheath.

The slider's ability to translate linearly in relation to the angulation structure can allow for simple and precise adjustment of the distance between the angulation structure and the ends of the sheaths during assembly. The linear translation mechanism facilitates fine-tuning of the tension in both control wires by symmetrically altering the position of the sheaths' ends relative to the angulation structure, effectively adjusting the length of the first control wire and the second control wire at a distal end of the insertion tube, e.g. an active bending section of the insertion tube, at the same time.

In some examples, the angulation assembly further comprises fixation means for maintaining a translation state of the slider with respect to the angulation structure.

The fixation means may include a locking mechanism, such as a clamp or a detent, that interacts with the slider to prevent unintended movement. In some examples, the slider is screwed or glued in place inside of the body in the selected tension state during assembly of an endoscope, thereby maintaining the desired tension in the control wires. In some examples, the fixation means include a friction-based locking mechanism, such as a set screw or a friction pad, which applies pressure to the slider to hold it in place.

In some examples, when the slider is pulled away from the angulation structure it induces a tension of the first control wire and the second control wire, when the angulation assembly is used in an endoscope.

In some examples, approaching the slider towards the angulation structure reduces a tension of the first control wire and the second control wire, when the angulation assembly is used in an endoscope.

Moving the slider closer to the angulation structure may effectively lengthen the control wires in an active bending section of the insertion tube, thus reducing tension in the control wires.

In some examples, the angulation assembly comprises a cylindrical shaft element extending along the rotation axis of the angulation structure, and the angulation assembly may further comprise a first pad for engaging the cylindrical protrusion to prevent rotation of the angulation structure.

The cylindrical shaft element may be provided as a cylindrical protrusion of the angulation structure protruding from the angulation structure along the rotation axis and/or as a section of a shaft connecting the angulation structure to the handle. The cylindrical shaft element can feature a cylindrical engagement surface which may serve as a mechanical interface that can be engaged to prevent or control the rotation of the angulation structure, thereby enhancing the stability and control of the angulation assembly. The first pad is designed to engage with the cylindrical shaft element, effectively preventing or increasing a friction involved with the rotation of the angulation structure when engaged. This interaction between the first pad and the cylindrical shaft element may provide a locking mechanism that can be used to stabilize the angulation structure during operation. The cylindrical shaft element may be integrated with the handle, e.g. along a mechanical interface between the handle and the angulation structure, for providing a compact (soft-) braking system. For example, the cylindrical shaft element may be provided on a shaft transmitting a torque from the handle onto the angulation structure and extending through a casing of the body, and may be e.g. arranged at an outer or at inner surface of the body.

In some examples, the angulation assembly comprises a second pad arranged to face an opposite face of the cylindrical shaft element with respect to the first pad and the first pad and the second pad are configured for engaging the cylindrical shaft element on opposite sides to prevent rotation of the angulation structure.

The first pad and the second pad engaging the cylindrical shaft element on opposite sides can exert opposing forces on the cylindrical shaft element, which may increase the reliability of the assembly, e.g. in view of manufacturing tolerances.

In some examples, the angulation assembly further comprises a rotatable actuator, the rotatable actuator and/or the cylindrical shaft element comprising a non-circular surface for supporting the first pad and/or the second pad between the rotatable actuator and/or the cylindrical shaft element, such that rotating the rotatable actuator modulates a distance between the cylindrical shaft element of the angulation structure and the rotatable actuator.

The non-circular surface may feature a spiraling engagement surface with a gradually changing radius, such that rotating the rotatable actuator with respect to the cylindrical shaft element may modulate an effective distance between the engagement surface and the cylindrical shaft element, e.g. for increasing/reducing the friction between the first pad and/or second pad and the cylindrical shaft element. The first pad and/or the second pad may be slidably mounted on the body, such that rotating the rotatable actuator with respect to the cylindrical shaft element may shift the position of the rotatable actuator, on which the first pad and/or the second pad are supported, for adjusting an effective bias of the first pad and/or the second pad onto the cylindrical shaft element. In some examples, the first pad and/or the second pad are mounted on the rotatable actuator to rotate with the rotatable actuator, and rotating the rotatable actuator shifts the location on the cylindrical shaft element at which the first pad and/or the second pad engage a non-circular surface of the cylindrical shaft element for adjusting an effective bias of the first pad and/or the second pad onto the cylindrical shaft element.

In an adjusted rotation state of the rotatable actuator, the angulation assembly may be soft braked, with the engagement of the pad(s) between the rotatable actuator and the cylindrical shaft element still enabling the angulation mechanism to be useable/moveable with a suitable operation force, while allowing a user to release the leaver while the active bending section maintains its adjusted position.

The rotatable actuator may support the first pad and/or the second pad on the engagement surface, which can allow for a flat and compact construction of the braking system. In some examples, the height of the pads along the rotation axis is smaller than 5 mm such as 3.5 mm or smaller. In some examples, the height of the soft brake mechanism including the pads and the rotatable actuator along the rotation axis is smaller than 5 mm such as 3.5 mm or smaller. The soft braking mechanism may be integrated with an angulation bearing of the angulation assembly, wherein the angulation bearing may be integrally formed in the body.

In some examples, the angulation structure further comprises a biasing element for biasing the first pad and/or the second pad towards the cylindrical shaft element.

The biasing element communicates with the first pad and the second pad, applying a force that pushes them towards the cylindrical shaft element. The biasing element may be a spring, an elastic material, or any other suitable mechanism that can exert a biasing force.

In some examples, the first pad and/or the second pad are arranged to face towards an outer surface of the cylindrical shaft element.

The cylindrical shaft element may protrude along the rotation axis of the angulation structure towards the handle, and may form part of the protrusion engaging the handle with the angulation structure. The cylindrical shaft element may be arranged at an outer surface of the body, and the first and/or the second pad may be arranged on the outer surface of the body for engaging the cylindrical shaft element.

The rotatable actuator may overlie and cover the first and/or the second pad on the outer surface of the body. The rotatable actuator may feature an annular shape arranged around the cylindrical shaft element and below the handle for enabling convenient access by an operator. An axially protruding feature may be provided on the rotatable actuator for facilitating rotating the rotatable actuator around the cylindrical shaft element. Thus, the rotatable actuator may be readily accessed while manipulating the handle. The rotatable actuator may be accessible and actuatable by the same finger (e.g. thumb) used to actuate the handle, for variably adjusting the amount of resistance against rotation. For example, both the handle and the rotatable actuator may be actuated by a human operator's thumb, when holding the angulation assembly with the same or opposite hand.

In some examples, an annular friction element is arranged on the cylindrical shaft element, and the first pad and/or the second pad engage the annular friction element to prevent rotation of the angulation structure.

The engagement may increase a friction between the first pad and/or the second pad and the cylindrical shaft element for preventing the angulation structure from rotating unintentionally. The pads and the annular friction element may be in physical contact for inducing a frictional force that stabilizes the angulation structure. The friction element may be pliable, e.g. for accommodating manufacturing tolerances of the mounting between the first pad and/or the second pad and the cylindrical shaft element, such as by providing a friction element made of rubber or rubber-like polymers, such as silicone or polyurethane.

In some examples, the annular friction element may be an elastic O-ring pulled onto the cylindrical shaft element. An elastic O-ring can provide a flexible yet firm frictional interface and may be easily assembled, e.g. by pulling the O-ring onto the cylindrical shaft element where the O-ring may be stretched into an elastically stretched state to conform to the shape of the cylindrical shaft element.

In some examples, the angulation structure further comprises an abutment feature which is configured to abut against a stopping post for limiting a rotation angle of the angulation structure. For example, the angulation structure may feature a protruding abutment portion extending parallel to the rotation axis from the angulation structure, and the body may comprise an integrally formed abutment post arranged to abut against the protruding abutment portion, when the angulation structure is turned towards a limit angle.

According to a second aspect, an assembly method for an endoscope comprises providing a body adapted to be held by a human operator and to be coupled to an insertion tube of the endoscope. An angulation assembly with an angulation structure is rotatably mounted inside the body. A first control wire and a second control wire are supported on the angulation structure. The first control wire and the second control wire extend from opposite sides of the angulation structure through the body to the insertion tube. Rotation of the angulation structure winds one of the first control wire and the second control wire and unwinds the other one of the first control wire and the second control wire. First ends of the first control wire and the second control wire are connected to a distal end of the insertion tube. Second ends of the first control wire and the second control wire are connected to the angulation structure. The first control wire and the second control wire are tensioned using a tensioning mechanism mounted in the body and coupled to the first control wire and the second control wire through a motion of the tensioning mechanism.

The body may comprise or be provided as two casing halves, with cylindrical protrusions and/or openings for aligning the rotation axis of the angulation structure. The angulation structure may be fitted onto a first casing half of the body, e.g. onto a cylindrical protrusion of the respective casing half and/or with an axial protrusion of the angulation structure extending from the angulation structure through an opening of the casing half of the body.

The control wires may be pulled over the angulation structure, e.g. into a channel of the angulation structure, such as to jointly extend from a coupling portion of the body around the angulation structure.

Tensioning the control wires using a tensioning mechanism mounted in the body and coupled to the wires through its motion ensures that the wires remain taut and responsive. This tensioning mechanism is beneficial as it maintains the optimal tension in the wires, preventing slack and ensuring precise control over the endoscope's movements.

Tensioning the control wires may be effected by rotation of a spindle type tensioning mechanism mounted on the angulation structure, with the control wires mounted on the spindle type tensioning mechanism and wound up to increase the tension of the first control wire and the second control wire. Additionally or alternatively, the control wires may be sleeved into respective wire sleeves held on a slider inside the body, and tensioning the control wires may be effected by displacement of the slider inside of the body.

With a screw type fastener as the spindle, the control wires may be connected to the screw type fastener, e.g. through inserting the control wires into a hole or into a recess of the screw type fastener, and may be fixed to the angulation structure by screwing the screw type fastener into the angulation structure, which may at the same time tension the control wires.

In some examples, the tube may also include the control wire, wherein the control wire may be arranged in said guiding elements of the tube elements.

The angulation assembly may be integrated with an interface/control portion of an endoscope and may, for example and without limitation, be configured for use in a bronchoscope, a sinuscope, a nasopharyngoscope, a laryngoscope, a laparoscope, a gastroscope, a duodeno-scope, a colonoscope, an echoendoscope, a hysteroscope, a cystoscope, a uroscope, a urethro-scope, a cardioscope, and an arthroscope. The dimensions of the angulation assembly may be chosen accordingly.

### LIST OF FIGURES

In the following, a detailed description of the present disclosure and examples thereof is given with reference to the figures. The figures show schematic illustrations of
Fig. 1: an endoscope according to an example of the present disclosure;
Fig. 2: an angulation assembly for an endoscope in accordance with an example of the present disclosure;
Fig. 3: another angulation assembly for an endoscope in accordance with an example of the present disclosure in a cross-sectional view;
Fig. 4A, 4B an angulation assembly for an endoscope in accordance with an example of the present disclosure in two perpendicular cross-sectional views;
Fig. 5A: an angulation assembly in accordance with an example of the present disclosure from a side view;
Fig. 5B: a soft braking assembly for an angulation assembly in accordance with an example of the present disclosure in a cross-sectional view; and
Fig. 6: a method in accordance with an example of the present disclosure in the form of a flow chart.

### DESCRIPTION OF EXAMPLES

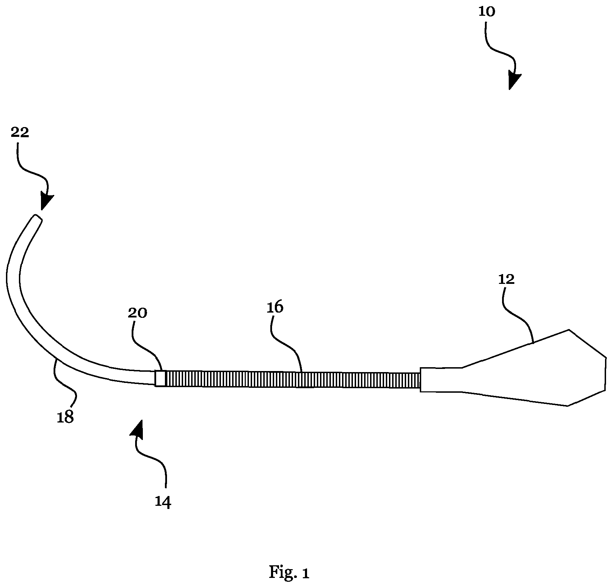

Fig. 1 shows a schematic illustration (not to scale) of an endoscope 10 according to an example of the present disclosure. The endoscope 10 includes an interface/control portion 12 and an insertion tube 14 comprising a passive bending section 16, and an active bending section 18, arranged distally with respect to the passive bending section 16, with a stop section 20 arranged between the active bending section 18 and the passive bending section 16. The endoscope 10 extends from the interface/control portion 12 to a distal end 22 arranged at a distal side of the active bending section 18.

The insertion tube 14 may be formed of different tube sections of a generally rigid material, which may be structured to control an effective bending stiffness of the insertion tube 14. A pipe made of stainless steel is particularly preferred, but other semi-rigid or rigid materials, e.g. titanium or a nickel-titanium alloy such as Nitionol may equally be used. Moreover, a pipe made of hard plastic can also be used. In principle, however, any material suitable for medical purposes can be used.

The insertion tube 14 may be substantially tubular shaped. The insertion tube 14 extends along a center line of the tube 14 from a proximal end adjacent to the interface/control portion 12 (e.g. connected or attached thereto via a connector) to a distal end 22, wherein the distal end 22 is facing away from the interface/control portion 12.

One or more ports (not shown) may be provided at the interface/control portion 12 and may for example include one or more fluid ports that are in fluid communication with an interior of the insertion tube 14, for example with a channel (not shown) formed by or arranged in the insertion tube 14. Additionally or alternatively, the one or more ports may include one or more light guide ports, wherein each of the light guide ports may for example be configured to receive a light guide (not shown) that is to be arranged within the insertion tube 14 and/or to provide coupling to a light guide (not shown) that is arranged within the insertion tube 14, for example in a channel formed by or arranged in the insertion tube 14. The interface/control portion 12 may further include one or more electrical contacts (not shown), e.g. to provide an electrical connection to the interior of the insertion tube 14, to a distal end 22 of the insertion tube 14 and/or to a distal tip (not shown) of the endoscope 10, which may e.g. be arranged at or connected to the distal end 22 of the insertion tube 14.

The interface/control portion 12 also include means for actuating a distal active bending section 18 of the insertion tube 14, for example one or more control knobs (not shown), which may e.g. be configured to apply tension to one or more control wires (not shown) coupled to the active bending section 18, e.g. via one or more attachment point at the distal end 22.

The interface/control portion 12 may be embodied as a single unit as shown in Fig. 1 or as two or more separate units, for example a control portion and an interface portion, wherein the control portion may e.g. be connected to the proximal end 22 of the insertion tube 14 via a connector and the interface portion may e.g. be connected to the control portion via a flexible tube or an umbilical cord.

The proximal passive bending section 16 may be configured to be bent or deformed when applying an external force. The passive bending section 16 may for example be made of (e.g. include) a flexible material. Additionally or alternatively, the passive bending section 16 may be made of a rigid or semi-rigid material such as metal (e.g. stainless steel) and may for example include a plurality of bendable segments, in each of which one or more openings may be provided in a wall of the insertion tube 14 to increase a flexibility of the insertion tube 14 in the respective bendable segment. In some examples, a proximal section of the insertion tube may also include a rigid section (not shown), either in addition to or instead of the passive bending section 16, e.g. at the proximal end adjacent to a connector to the interface/control portion. A length and a diameter of the insertion tube 14 may be chosen to be suitable for the type of endoscope that the insertion tube 14 is to be used with. The insertion tube 14 may for example have a length between 20 cm and 200 cm, in some examples between 50 cm and 150 cm. An outer diameter of the tube 14 may for example be between 1 mm and 20 mm, in some examples between 2 mm and 10 mm.

In the example of Fig. 1, an active bending section 18 is provided which can be actively angulated based on a controlling force applied through the interface/control portion 12, in particular via control wires passing from the interface/control portion through the passive bending section 16 to the active bending section 18. The default configuration of the active bending section 18 may for example be a straight configuration, in which the insertion tube 14 is not bent and the center line is a straight line. The default configuration may for example be the configuration that the insertion tube 14 ideally (e.g. without any external perturbation) assumes when no additional tension is applied to the control wires, e.g. such that the control wires create a uniform (non-directional) compressive tension in the longitudinal direction. In some examples, the active bending section 18 may be actuated or controlled actively, e.g. using corresponding means for actuating the active bending section 18 such as control wires. When the control wires are tensioned, the active bending section 18 may assume a bent configuration, with the center line following a curved line between a proximal and a distal end of the active bending section 18, as shown in Fig. 1. The control wires may be configured as Bowden cables, and a cable sleeve of the control wires may extend from the interface/control portion 12 of the endoscope 10 to the stop section 20 for limiting a deformation of the passive bending section 16 in response to a tensioning of the control wires. The stop section 20 may comprise lateral mechanical stops for preventing movement of the respective ends of the cable sleeves, e.g. by defining an axial abutment for an end of the respective cable sleeve on opposite sides of the insertion tube 14, when viewed along the longitudinal direction.

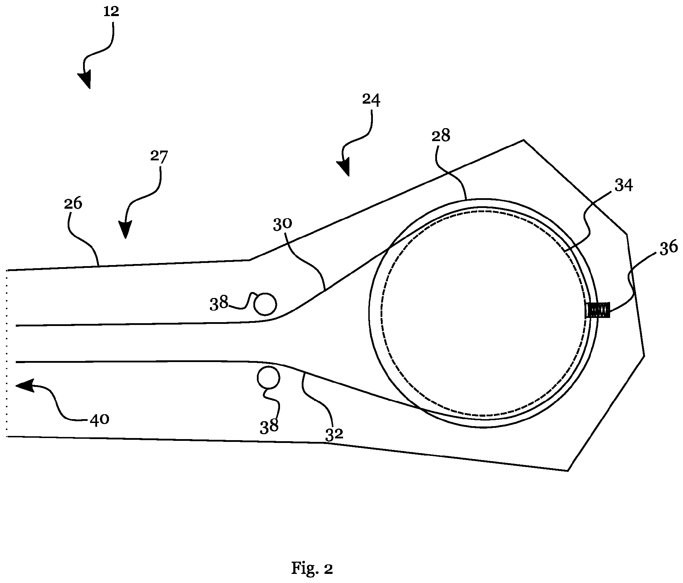

Fig. 2 illustrates a partial cross-sectional view of an angulation assembly 24 for an interface/control portion 12 of an endoscope 10 according to an example. The angulation assembly 24 comprises a body 26 with a grip portion 27 for holding the body 26 when using the endoscope 10. The body 26 is adapted to be held by a human operator and is designed to be coupled to an insertion tube 14 of the endoscope 10. The body 26 may be the structural body of the interface/control portion 12, and may be formed of a polymer, e.g. as an injection molded part. The body 26 may comprise two casing halves, e.g. configured to act as opposite sides of the body 26, which can be joined to each other along the normal of the plane of projection of Fig. 2 to assemble opposite sides of the body 26.

The body 26 rotatably mounts an angulation structure 28 implemented as a wheel mounted inside the body 26, with the rotation axis of the angulation structure 28 arranged along the normal of the plane of projection of Fig. 2. The angulation structure 28 is configured to support a first control wire 30 and a second control wire 32. The first control wire 30 and the second control wire 32 run along the perimeter of the angulation structure 28 inside of a channel 34 inside a radial face of the angulation structure 28. The first control wire 30 and the second control wire 32 both connect to a mounting and tensioning structure 36 mounted on the angulation structure 28, the mounting and tensioning structure 36 being implemented as a spindle in the example of Fig. 2. The first control wire 30 and the second control wire 32 extend from opposite sides of the angulation structure 28 past respective deflection posts 38 inside of the body 26 towards a coupling portion 40 of the body 26, which may couple the angulation assembly 24 to an interface (not shown) with an insertion tube 14 of the endoscope 10.

By rotating the angulation structure 28 inside the body 26, one of the first control wire 30 and the second control wire 32 is tensioned, while the other one of the first control wire 30 and the second control wire 32 is relaxed, such that rotating the angulation structure 28 allows for controlling a bending motion of an insertion tube 14 coupled to the interface/control portion 12 to achieve a desired angulation state of the insertion tube 14.

The mounting and tensioning structure 36 on the angulation structure 28, implemented as a spindle in Fig. 2, acts as a tensioning mechanism for the first control wire 30 and the second control wire 32, and is configured to adjust the tension state of the control wires 30, 32 through a motion of the tensioning mechanism. This spindle is coupled to both the first control wire 30 and the second control wire 32. The spindle can be rotated to wind up the first control wire 30 and the second control wire 32 at the same time for symmetrically tensioning the first control wire 30 and the second control wire 32. In some examples, the first control wire 30 and the second control wire 32 are joined to each other, e.g. are part of the same wire, and the first control wire 30 and the second control wire 32 may be sections of the joined wire extending from opposite sides of the angulation structure 28 towards the coupling portion 40. The first control wire 30 and the second control wire 32 when joined to each other may be connected to the mounting and tensioning structure 36 by routing the first control wire 30 and the second control wire 32 through a recess or aperture of the spindle, e.g. a notch in the spindle or a through hole running through the spindle, and by rotating the spindle to wind up the first control wire 30 and the second control wire 32 onto the spindle.

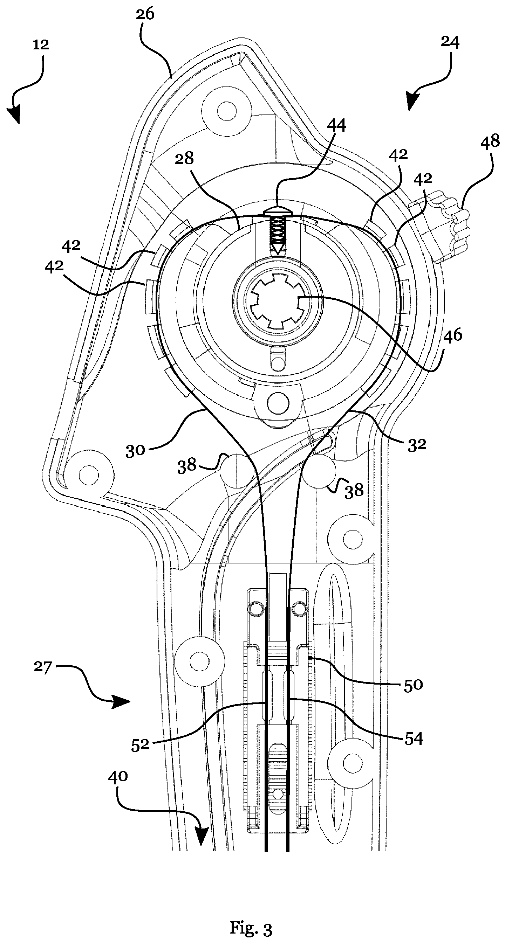

Fig. 3 illustrates a partial cross-sectional view of an angulation assembly 24 for an interface/control portion 12 of an endoscope 10 according to another example similar to the example illustrated in Fig. 2 and with the plane of projection also being normal to the rotation axis of the angulation structure 28. The cross-sectional view shows a first casing half of the body 26 which can be closed with a matching second casing half (not shown).

Similar to the example of Fig. 2, the body 26 houses an angulation structure 28 which is rotatably mounted inside of the body 26. The angulation structure 28 comprises a plurality of radially protruding webs 42 delimiting a control wire path with respect to the axial direction of the angulation structure 28 (i.e. along the rotation axis of the angulation structure 28).

A first control wire 30 and a second control wire 32 run along the radial perimeter of the angulation structure 28 between the webs 42 and extend from opposite sides of the angulation structure 28 towards a coupling portion 40 of the body 26, such that rotating the angulation structure 28 winds up one of the control wires 30, 32 and unwinds the other one of the control wires 30, 32, effectively allowing to tension either the first control wire 30 or the second control wire 32.

In some examples, the webs 42 comprise axially projecting top sections, which may further limit a radial motion of the control wires 30, 32. For example, the webs 42 may feature a substantially L-shaped cross-section, when viewed along the circumferential direction of the angulation structure 28, to form a C-shaped seating with the radial outer surface of the angulation structure 28 for the control wires 30, 32, and the L-shaped webs 42 may be mirrored in an alternating fashion to effectively form a control wire channel between two neighboring webs 42 along the radial outer surface of the angulation structure 28, delimiting a control wire path on all sides, but enabling insertion of the control wires 30, 32 into the control wire channel from the side, e.g. when not under tension.

The control wires 30, 32 are both coupled to a spindle-type mounting and tensioning structure 36 implemented as a screw-type fastener 44 which is screwed into the angulation structure 28 at a mounting side opposite the side facing the coupling portion 40, the mounting side featuring a recess for receiving the screw type fastener 44. The screw type fastener 44, when rotated in a tensioning direction, winds both control wires 30, 32 onto the screw type fastener 44, thereby increasing their tension. The first and second control wires 30, 32 may be threaded through a recess or aperture in the screw type fastener 44 (e.g. a through hole above or in a threaded section of the fastener), ensuring they are securely held and can be tensioned effectively and evenly. At the same time, the screw type fastener 44 can fixedly mount the control wires 30, 32 on the angulation structure 28, allowing for a compact and simple assembly of the angulation structure 28 and the control wires 30. As shown in Fig. 3, portions of the control wires 30, 32 may be received in the recess of the mounting side, when the control wires 30, 32 are mounted on the angulation structure 28 and tensioned.

The angulation structure 28 comprises a central shaft slot 46 arranged at and extending into the angulation structure 28 along the rotation axis of the angulation structure 28. The cross section of the central shaft slot 46, when viewed along the rotation axis of the angulation structure 28, features a notched perimeter for enabling a form fitting engagement with a matching protrusion of a shaft (not shown) for coupling the angulation structure 28 inside the body 26 to a handle 48 arranged outside the body 26.

In Fig. 3 the handle 48 comprises a lever portion (not shown) extending perpendicularly from the shaft outside the body 26 and a bracket portion extending outside the body 26 along the rotation axis between opposite sides of the body 26. Displacement of the handle 48 induces rotation of the angulation structure 28 for operating the control wires 30, 32.

The angulation assembly 24 further comprises a slider 50 arranged between the angulation structure 28 and the coupling portion 40 which is part of the tensioning mechanism of the angulation assembly 24. The slider 50 has mounting points for fixedly holding proximal ends of a first sheath 52 and a second sheath 54, which sheath the first control wire 30 and the second control wire 32, respectively. The control wires 30, 32 extend from the angulation structure 28 towards the slider 50 and into the respective sheaths 52, 54.

The sheaths 52, 54 extend through a portion of the insertion tube 14 towards respective distal ends of the sheaths 52, 54, which are mounted at respective mounting features at the stop section 20 of the insertion tube 14. The mounting features for the distal ends of the sheaths 52, 54, may be spaced from each other along the radial direction of the insertion tube 14, such as to define respective radially spaced mounts for the first and second control wires 30, 32 at the stop section 20 for guiding a compressive tension along the control wires 30, 32 to bend the active bending section 18 of the insertion tube 14.

The slider 50 is configured for linear translation with respect to the angulation structure 28, allowing for the adjustment of the distance between the angulation structure 28 and the proximal ends of the sheaths 52, 54. Approaching the slider 50 towards the angulation structure 28 reduces a length of a control wire section between the slider 50 and the angulation structure 28 and increases an effective length of a control wire section between the distal ends of the sheaths 52, 54 and a distal attachment point of the control wires 30, 32 closer to the distal end 22 of the insertion tube 14 for both of the control wires 30, 32. Accordingly, approaching the slider 50 towards the angulation structure 28 may reduce the tension of the control wires 30, 32 when the assembly 24 is used in an endoscope 10. Conversely, distancing the slider 50 and the angulation structure 28 through a linear translation of the slider 50 in the body 26 may increase an effective tension of the control wires 30, 32, when the distal ends of the control wires 30, 32 are fixedly connected to a distal attachment point of the insertion tube 14, and the distal ends of the sheaths 52, 54 are fixed at the stop section 20 of the insertion tube 14.

Fixation means may be included to maintain the translation state of the slider 50 with respect to the angulation structure 28. For example, the slider 50 may feature an oblong slot for fixedly connecting the slider 50 and the body 26 in a desired linear translation state of the slider 50 inside of the body 26, e.g. with a fastener extending through the slot and clamping the slider 50 against the body in a desired linear translation state along the slot. As another example, the angulation assembly 24 may comprise a clamping bracket (not shown) which may be brought into force locking engagement with the slider 50 when attached to the body 26, e.g. by connecting the clamping bracket to the body 26 with suitable fasteners such that the clamping bracket clamps the slider 50, e.g. against a portion of the body 26.

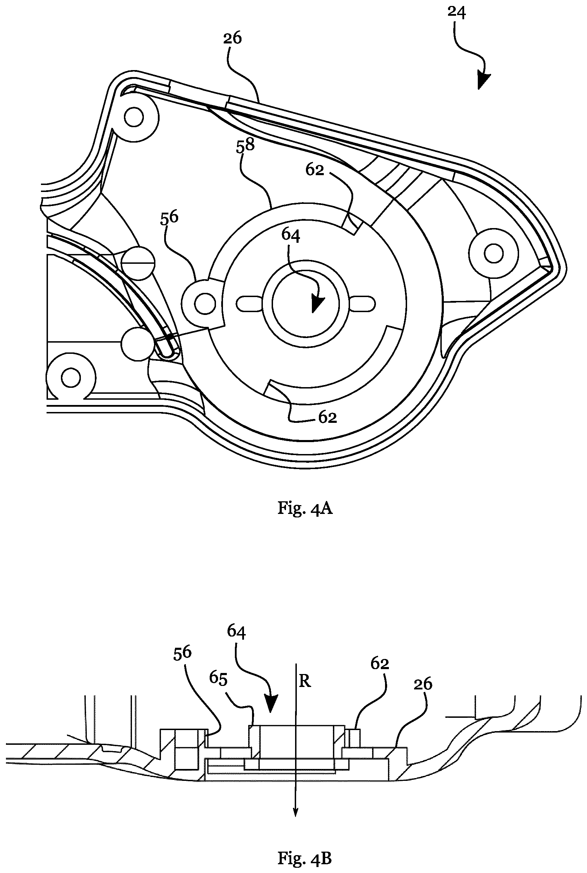

Fig. 4A illustrates a further partial cross-sectional view of the angulation assembly 24 for an interface/control portion 12 of an endoscope 10 as shown in the example of Fig. 3. The cross section is taken close to a seating of the angulation structure 28 on the body 26. Fig. 4B illustrates a cross-sectional view of the angulation mechanism 24 with the plane of projection perpendicular to the cross-sectional view of Fig. 4A and along the rotation axis R of the angulation structure 28.

The angulation assembly 24 comprises a stopping post 56 protruding from the body 26 towards the angulation structure 28. The illustrated example, the stopping post 56 is integrally formed with the body 26 and integrated with a substantially circular seating recess 58 in the body 26 for receiving a rotation control structure connected to the angulation structure 28.

The rotation control structure may be integrally formed with the angulation structure 28 and may protrude from the angulation structure 28 parallel to the rotation axis of the angulation structure 28. The rotation control structure protrudes into the seating recess 58 and the seating recess 58 may delimit a seat for the rotation control structure in the radial direction. The rotation control structure is rotatably mounted in the seating recess 58 and comprises an annular section portion 62 configured to abut against the stopping post 56 to stop rotation of the angulation structure 28 past a pre-defined rotation angle. As shown in Fig. 4, two annular section portions 62 may be provided in different sections about the rotation axis of the angulation structure 28 and may abut against opposite sides of the stopping post 56 in the circumferential direction around the rotation axis of the angulation structure 28 for defining rotation limits of the angulation structure 28 in the body 26.

The rotation control structure is aligned with a through hole 64 extending through the body 26 at the rotation axis of the angulation structure 28 for enabling a shaft to extend from the angulation structure 28 through the body 26 towards a handle 48 of the angulation assembly 24. The body 26 may feature circular bearing walls 65 integrally molded with the casing half of the body 26 to protrude towards angulation structure 28 and arranged around the rotation axis for engaging with a circular recess of the angulation structure 28 to rotatably mount the angulation structure 28 on the body 26.

Providing the rotation control structure on the angulation structure 28 may enable the adjustment of the rotation angle limits of the angulation assembly 24 through the exchange of the angulation structure 28 or a rotation control structure mounted on the angulation structure 28, such that the same body 26 may be used for different applications of the angulation assembly 24, e.g. requiring different rotation limits for the angulation structure 28. The rotation angle limits may be adjusted based on modifications to the rotation control structure, such as the width and/or position of the annular section portions 62 along the circumferential direction. In some examples, multiple stopping posts 56 are provided on the body 26. In some examples, the stopping posts 56 are provided to protrude from and rotate with the angulation structure 28, and the seating recess 58 of the body 26 comprises annular section portions 62 protruding radially towards the rotation axis to abut against a stopping post 56 on the angulation structure 28. Additionally or alternatively, the angulation structure 28 may feature a stopping pillar for abutting against a stopping post 58 of the body 58 or protruding into an annular section recess of the body 26 for delimiting the rotation angle of the angulation structure 28 mounted in the body 26.

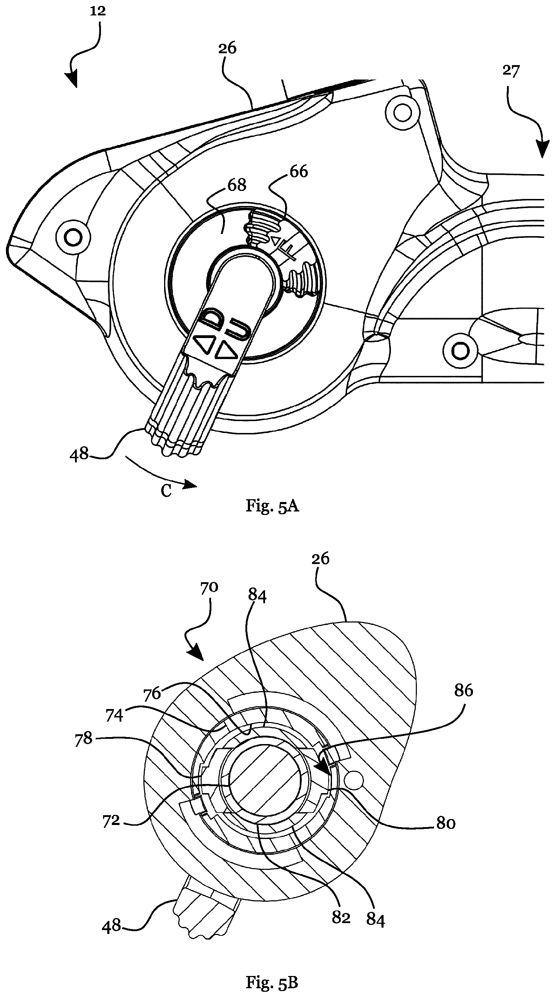

Fig. 5A illustrates a side view of an angulation assembly 24 of an interface/control portion 12 of an endoscope 10 showing the outside of the body 26 of the angulation assembly 24 according to an example. The body 26 comprise a grid portion 27 for holding the interface/control portion 12 arranged distantly (i.e. towards a coupling portion 40 for an insertion tube 14) with respect to an angulation structure 28 (not shown in Fig. 5A) of the angulation assembly 24.

The angulation mechanism 24 comprises a handle 48 arranged on the outside of the body 26 and comprising a shaft portion (not shown in figure 5A) extending along the rotation axis R of the angulation structure 28 through the body 26 to connect the angulation structure 28 to the outside of the body 26 for controlling the angulation assembly 24. The handle 48 further comprises a bracket portion which extends around the body 26 at least partially along a direction parallel to the rotation axis R, but space with respect to the rotation axis R of the angulation structure 28 along the radial direction. An operator may hold the body 26 at the grid portion 27 and operate the angulation assembly 24 by displacing the handle 48 along the circumferential direction C to adjust a relative tensioning state of the first control wire 30 and the second control wire 32 for actively angulating an active bending section 18 of an insertion tube 14 (not shown in figure 5A). As shown in figure 5A, the handle 48may be in a neutral position where the bending section 18 is approximately straight, and the handle 48 may then be rotated by the user in both the positive and negative circumferential direction C for actuating the bending section 18 in the desired corresponding (positive/negative) direction.

The angulation assembly 24 further comprises a braking handle 66 configured to be rotatable about the rotation axis R to adjust a friction state of the angulation assembly 24, such as to control a force required to change the relative tensioning state of the first control wire 30 and the second control wire 32. The braking handle 66 protrudes from an annular cover 68 extending around the rotation axis R for shielding a braking assembly. The braking handle 66 may be integrally formed with the annular cover 66 as part of a rotatable actuator for adjusting the friction associated with rotating the angulation structure 28. The rotatable actuator may be shaped to be actuatable by the same thumb or finger used to actuate the handle 48.

Figure 5B shows a partial cross-sectional view of the angulation assembly 24 along the same view axis as figure 5A illustrating functional parts of a braking assembly 70 according to an example.

The braking assembly 70 comprises a cylindrical shaft element 72 extending along the rotation axis R (along the normal of the plane of projection of figures 5A, 5B) connecting the angulation structure 28 to the handle 48. The braking assembly 70 further comprises a braking control structure 74 arranged around a perimeter of the cylindrical shaft element 72 and featuring a noncircular inner biasing surface 76 facing an outer surface of the cylindrical shaft element 72 along the radial direction.

A first pad 78 and a second pad 80 of the braking assembly 70 are arranged between the noncircular inner biasing surface 76 of the braking control structures 74 and the outer surface of the cylindrical shaft element 72 on opposite sides of the cylindrical shaft element 72. The cylindrical shaft element 72 is configured to be engaged by the first pad 78 and the second pad 80 to prevent unwanted rotation of the angulation structure 28. In the illustrated example, the first pad 78 and the second pad 80 are configured to abut against a friction element 82 arranged on the outer perimeter of the cylindrical shaft element 72. The friction element 82 may be implemented as an O-ring pulled onto the cylindrical shaft element 72 to lie between the outer surface of the cylindrical shaft element 72 and the first pad 78 and the second pad 80. The first pad 78 and the second pad 80 are slidably held between circumferential abutment posts 84, which prevent movement of the first pad 78 and the second pad 80 in the circumferential direction C, such as to limit a motion of the first pad 78 and the second pad 80 to a linear sliding motion towards/away from the cylindrical shaft element 72.

The circumferential abutment posts 84 may prevent rotation of the first pad 78 and the second pad 80, such that a current engagement portion of the noncircular inner biasing surface 76 of the braking control structure 74 abutting against an outer side of the first pad 78 and the second pad 80 shifts as a function of the rotation of the braking control structure 74.

The braking control structure 74 is connected to the braking handle 66, such that rotating of the braking handle 66 around the rotation axis R by an operator rotates the braking control structure 74 around the cylindrical shaft element 72. For example, the braking control structure may be integrally formed with the annular cover 68 and the braking handle 66.

As shown in the example of figure 5B, the noncircular inner biasing surface 76 of the braking control structure 74 may feature a spiraling cross-sectional portion, in which the radius from the rotation axis R smoothly varies, when viewed along the rotation axis R, such that rotation of the rotation control structure 74 may smoothly shift the current engagement portion of the non-circular inner biasing surface 76 abutting against the first pad 78 and the second pad 80, which may vary an effective distance between the non-circular inner biasing surface 76 and the outer surface of the circular shaft element 72. Varying the distance between the non-circular inner biasing surface 76 and the outer surface of the circular shaft element 72 may drive the first pad 78 and the second pad 80 towards (away from) the outer surface of the circular shaft element 72 such that the first pad 78 and the second pad 80 are (less) forced against the friction element 82 arranged on the outer surface of the circular shaft element 72. The operation of the braking control structure 74 enables a fine tuning of the effective friction associated with operating the angulation assembly 24, which can be used to adapt the angulation assembly 24 during operation both in terms of a passive response of the insertion tube 14 to external forces as well as to tune active control of the insertion tube 14 by an operator, e.g. for preventing inadvertent actuation or for fine control of a bending state of the active bending section 18 of the insertion tube 14.

The braking control structure 74 may be integrally formed with the handle 66 for forming a rotatable actuator with a non-circular surface which can be actuated from an outer side of the rotatable actuator, and which can support the first and/or second pad 78, 80 on an opposite side, facing the body 26, for modulating the distance between the cylindrical shaft element 72 and the pads 78, 80 upon rotation of the rotatable actuator. When the braking handle 66 is rotated about the rotation axis R, the pads 78, 80 are forced into engagement with the annular friction element 84 to prevent rotation of the angulation structure 28 with varying force thresholds. Rotating the rotatable actuator may effectively displace the contact point between the pads 78, 80 and the braking control structure 74 towards a different section of the braking control structure 74 for adjusting the effective distance between the surface of the cylindrical shaft element 72 and the surface of the braking control structure 74 mounting the pads 78, 80. The braking handle 66 may be an integrally molded protrusion extending away from the body 26, and the rotatable actuator may be turned around the cylindrical shaft element 72 beneath the handle 48 to adjust the friction state.

The rotation control structure 74 may further comprise a non-engagement segment 86 of the inner surface of the rotation control structure 74, wherein the non-engagement segment 86 is recessed from the circular shaft element 72, such that a distance between the friction element 84 and the inner biasing surface of the rotation control structure 74 is similar to or greater than an extent of the first pad 78 and the second pad 80 in the radial direction, such that a friction associated with rotation of the circular shaft element 72 is reduced with respect to a state, in which the first pad 78 and the second pad 80 are supported by the inner biasing surface 76. In the circumferential direction, the inner surface of the rotation control structure 74 may comprise a stepped portion between the non-circular inner biasing surface 76 and the non-engagement segment 86 such as to define a haptic feedback to an operator when the rotation control structure 74 is rotated from a non-engagement state, in which the first pad 78 and the second pad 80 are supported by the non-engagement segment(s) 86, towards an engagement state, in which the first pad 78 and the second pad 80 are supported by the non-circular inner biasing surface(s) 76, and back.

In the illustrated example, the non-engagement segment 86 is delimited in the circumferential direction by two stepped portions of different height in opposite directions, such as to provide different force thresholds for rotating the rotation control structure 74 towards two different engagement states, in which the first pad 78 and the second pad 80 are supported by respective non-circular inner biasing surfaces 76 on opposite sides of the non-engagement segment 86, e.g. effectively preventing rotation in a first direction and providing haptic feedback in the opposite direction, when the rotation control structure 74 is rotated past a state, where a radial outer edge of the first pad 78 and/or the second pad 80 abuts against the stepped portion delimiting the non-engagement segment 86 in the respective direction.

In the illustrated example, the first pad 78 and/or the second pad 80 comprise a radially inner portion featuring a concave abutment surface conforming to a perimeter of the friction element 84/the circular shaft element 72 and configured for frictional engagement with the friction element 84/the circular shaft element 72 at a radially inner side of the first pad 78 and/or the second pad 80. The first pad 78 and/or the second pad 80 may further comprise a radially outer support portion configured to support the first pad 78 and/or the second pad 80 on a radially inner surface of the rotation control structure 74, e.g. on the non-engagement segment(s) 86 or on the non-circular inner biasing surface 76, and for abutting against the stepped portions of the rotation control structure 74 in the circumferential direction as the rotation control structure 74 in is rotated. The radially outer support portion may feature a smaller extent in the circumferential direction than the concave abutment surface, and may be provided as a support stud, as shown in Fig. 5B.

The skilled person will appreciate that the friction element 84 is an optional element, and different examples may feature a direct engagement between the circular shaft element 72 and the first pad 78 and/or the second pad 80, and/or a friction element can be provided on the concave abutment surface of the first pad 78 and/or the second pad 80 for increasing a friction when the first pad 78 and/or the second pad 80 abut against the circular shaft element 72. The skilled person will further appreciate that the braking assembly may be provided with a single pad 78/80 or more than two pads 78/80 in different examples.

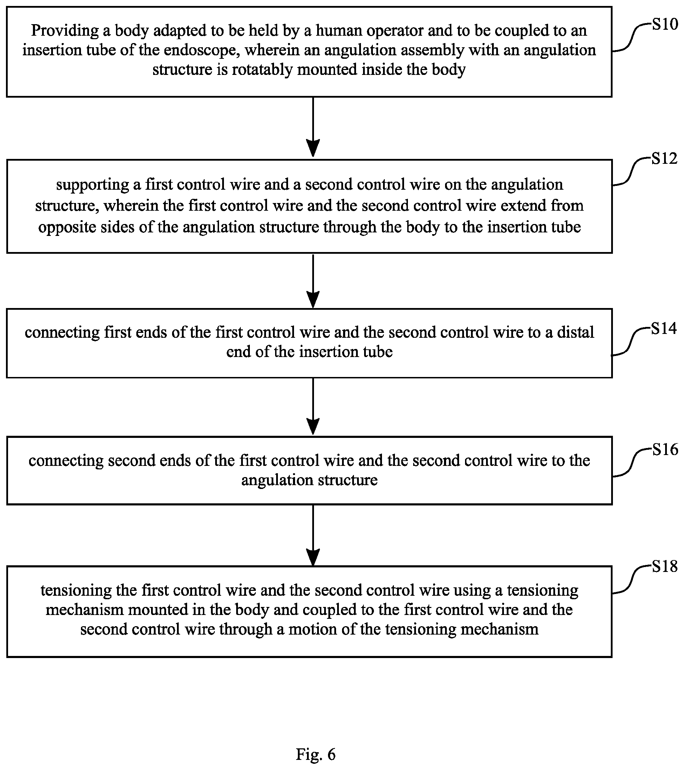

Fig. 6 illustrates a flow chart for a method of assembling an angulation mechanism 24 according to an example. The method comprises providing a body 26 adapted to be held by a human operator and to be coupled to an insertion tube 14 of the endoscope 10, wherein an angulation assembly 24 with an angulation structure 28 is rotatably mounted inside the body 26 (S10). The method further comprises supporting a first control wire 30 and a second control wire 32 on the angulation structure 28, wherein the first control wire 30 and the second control wire 32 extend from opposite sides of the angulation structure 28 through the body 26 to the insertion tube 14 (S12), such that rotation of the angulation structure 28 winds one of the first control wire 30 and the second control wire 32 and unwinds the other one of the first control wire 30 and the second control wire 32. The method further comprises connecting first ends of the first control wire 30 and the second control wire 32 to a distal end 22 of the insertion tube 14, and connecting second ends of the first control wire 30 and the second control wire 32 to the angulation structure 28. The method further comprises tensioning the first control wire 30 and the second control wire 32 using a tensioning mechanism mounted in the body 26 and coupled to the first control wire 30 and the second control wire 32 through a motion of the tensioning mechanism.

The body 26 may comprise first and second casing halves, which may be joined to each other for providing a closed body 26, e.g. for housing the angulation structure 28 and the control wires 30, 32. The assembly of the angulation assembly 24 may begin with the provision of a first casing half of the body 26, on which the angulation structure 28 is rotatably mounted. For example, the body 26 may feature a (circularly) cylindrical protrusion protruding towards an interior of the body 26, and the angulation structure 28 may feature a corresponding recessed portion, or vice versa, for defining a from fitting engagement of the angulation structure 28 inside the body 26 such that the angulation structure 28 is rotatably mounted to rotate about a rotation axis R.

The control wires 30, 32 may be sheathed in corresponding first and second sheaths 52, 54, which may be joined to a slider 50 slidably supported on the first casing half of the body 26. The control wires 30, 32 may be inserted into a recess or aperture of a mounting and tensioning structure 36, such as a through hole of a screw type fastener 44, in a state connected to or disconnected from the angulation structure 28. The control wires 30, 32 may be arranged on the angulation structure 28 to extend from opposite sides towards respective ends of the sheaths 52, 54. The control wires 30, 32 may extend through the sheaths 52, 54, whose ends may be held at a stop section 20 of an insertion tube 14 connected to the body 26. The control wires 30, 32 may be connected to mounting points at the distal end 22 of the insertion tube 14 with their first ends, and the mounting and tensioning structure 36 may be connected to the angulation structure 28 with their respective second ends. The mounting and tensioning structure 36 may subsequently be used to tension the control wires 30, 32, e.g. by rotating a spindle-type mounting and tensioning structure 36 on the angulation structure 28, such as a screw type fastener 44. A tension of the control wires 30, 32 may subsequently be fine tuned by linearly displacing the slider 50 inside of the body 26 to adjust a distance between the ends of the sheaths 52, 54 and the angulation structure 28.

The body 26 may be closed with a second casing half of the body 26, which matches the first casing half of the body 26, for enclosing the angulation structure 28 inside the body 26 from opposite sides. The angulation structure 28 may be connected to a handle 48 on the outside of the body 26, e.g. by inserting a shaft portion of the handle 48 into a corresponding opening of the body 26 which may be coaxial with a rotation axis R of the angulation structure 28. For example, the shaft portion of the handle 48 may be brought into form fitting engagement with the angulation structure 28 by providing corresponding form fitting shapes allowing to transmit a torque from the handle 48 applied outside the body 26 to the angulation structure 28 inside the body 26. The body 26 may comprise corresponding openings on opposite sides of the body 26, allowing to engage and connect corresponding shaft portions to the angulation structure 28 from opposite sides.

The skilled person will appreciate that the order of the steps provided in Fig. 6 is exemplary and that a different order of the steps may even be advantageous depending on the specifics of the example. For example, the control wires 30, 32 may be first connected to respective mounting points at the distal end 22 of the insertion tube 14, before the control wires 30, 32 are supported on the angulation structure 28. Converesely, in some examples, the second ends of the control wires 30, 32 may be mounted to the angulation structure 28 prior to attachment of the first ends of the control wires 30, 32 to the distal end 22 of the insertion tube 14.

In some examples, the control wires 30, 32 are initially loosely connected to the angulation structure 28 with a screw-type fastener 44 and subsequently have their respective first ends connected to the distal end 22 of the insertion tube 14. The screw-type fastener 44 may then be tightened, winding up the control wires 30, 32, thereby tensioning the control wires 30, 32 in a state, where the control wires 30, 32 are supported on the angulation structure 28 to extend from opposite sides towards the coupling portion 40.

The skilled person will appreciate that the handle 48 may also be implemented as a rotatable knob, may only be accessible on one side of the body 26, or may be omitted in favor of an (electrical) actuator or gear box, e.g. arranged inside the body 26.

Moreover, although an example is shown of interface control portion 12 with a single angulation assembly 24 for articulating an insertion tube 14 in a single articulation plane, the insertion tube 14 may equally be configured for active bending in two different articulation planes, e.g. for articulation in two perpendicular directions, and the body 26 may be configured to mount a further angulation assembly 24 for controlling a second pair of control wires 30, 32 for controlling an active bending of the insertion tube 14 in a second articulation plane.

The second pair of control wires 30, 32 may extend through the insertion tube 14 alongside the other (first) pair of control wires 30, 32 up to the stop section 20, where the ends of the respective sheaths 52, 54 may be arranged in the second articulation plane. In the body 26, the second pair of control wires 30, 32 may extend in the same plane as the other pair of control wires 30, 32, in particular when the control wires 30, 32 are sheathed in respective sheaths 52, 54.

In the active bending section 18 of the insertion tube, the control wires 30, 32 may be guided through corresponding guiding elements of the insertion tube 14 for transmitting a compressive force along the control wires 30, 32 onto the insertion tube 14 to bend the active bending section 18 in response to a rotation of the angulation structure 28.

The examples of the present disclosure disclosed herein only constitute specific examples for illustration purposes. The present invention can be implemented in various ways and with many modifications without altering the underlying basic properties. Therefore, the present invention is only defined by the claims as stated below.

## Claims

1. An angulation assembly (24) for an endoscope (10), the angulation assembly (24) comprising
a body (26) adapted to be held by a human operator and to be coupled to an insertion tube (14) of the endoscope (10),
an angulation structure (28) rotatably mounted inside the body (26), the angulation structure (28) being configured for supporting a first control wire (30) and a second control wire (32), the first control wire (30) and the second control wire (32) extending from opposite sides of the angulation structure (28) through the body (26) to the insertion tube (14), such that rotation of the angulation structure (28) winds one of the first control wire (30) and the second control wire (32) and unwinds the other one of the first control wire (30) and the second control wire (32);
a tensioning mechanism mounted in the body (26) in a selected tensioning state coupled to the first control wire (30) and the second control wire (32), wherein a motion of the tensioning mechanism adjusts the tensioning state to increase a tension of the first control wire (30) and the second control wire (32), when coupled to the insertion tube (14).

2. The angulation assembly (24) of claim 1, further comprising a handle (48) accessible from the outside of the body (26) and wherein displacement of the handle (48) induces a rotation of the angulation structure (28), wherein the handle (48) in particular comprises a protrusion protruding into the body (26) towards the angulation structure (28), wherein the protrusion protrudes along a rotation axis of the angulation structure (28), wherein the angulation structure (28) and the handle (48) preferably are in form-fitting engagement.

3. The angulation assembly (24) of claim 1 or 2, wherein the tensioning mechanism comprises a spindle (36, 44) mounted on the angulation structure (28) and coupled to the first control wire (30) and the second control wire (32), wherein rotation of the spindle (36, 44) in a tensioning direction winds up both the first control wire (30) and the second control wire (32) onto the spindle (36, 44), wherein the spindle (36, 44) is in particular a screw type fastener (44), wherein the screw type fastener (44) is screwed into the angulation structure (28) for increasing the tension of the first control wire (30) and the second control wire (32).

4. The angulation assembly (24) of claim 3, wherein the first control wire (30) and the second control wire (32) are threaded through a recess or aperture (46) of the spindle (36, 44).

5. The angulation assembly (24) of any one of the preceding claims, wherein the first control wire (30) and the second control wire (32) are joined to each other.

6. The angulation assembly (24) of any one of the preceding claims, wherein the tensioning mechanism comprises a slider (50) comprising mounting points for fixedly holding ends of a first sheath (52) and a second sheath (54) sheathing the first control wire (30) and the second control wire (32), respectively, wherein the slider (50) is configured for linear translation with respect to the angulation structure (28) to adjust a distance between the angulation structure (28) and the ends of the first sheath (52) and the second sheath (54).

7. The angulation assembly (24) of claim 6, wherein the angulation assembly (24) further comprises fixation means for maintaining a translation state of the slider (50) with respect to the angulation structure (28).

8. The angulation assembly (24) of claim 6 or 7, wherein when the slider (50) is pulled away from the angulation structure (28) a tension of the first control wire (30) and the second control wire (32) is induced, when the angulation assembly (24) is used in an endoscope (10).

9. The angulation assembly (24) of any one of the preceding claims, wherein the angulation assembly (24) comprises a cylindrical shaft element (72) extending along a rotation axis of the angulation structure (28), and wherein the angulation assembly (24) further comprises a first pad (78) for engaging the cylindrical shaft element (72) to prevent rotation of the angulation structure (28).

10. The angulation assembly (24) of any one of the preceding claims, wherein the angulation assembly (24) comprises a second pad (80) arranged to face an opposite face of the cylindrical shaft element (72) with respect to the first pad (78), wherein the first pad (78) and the second pad (80) are configured for engaging the cylindrical shaft element (72) on opposite sides to prevent rotation of the angulation structure (28).

11. The angulation structure (28) of claim 9 or 10, wherein the angulation assembly (24) further comprises a rotatable actuator (74), the rotatable actuator (74) and/or the cylindrical shaft element (72) comprising a non-circular surface (76) for supporting the first pad (78) and/or the second pad (80) between the rotatable actuator (74) and/or the cylindrical shaft element (72), such that rotation of the rotatable actuator (74) modulates a distance between the cylindrical shaft element (72) of the angulation structure (28) and the rotatable actuator;
wherein the rotatable actuator is in particular accessible and actuatable by a same finger of an operator that accesses the handle (48), for variably adjusting an amount of resistance against rotation.

12. The angulation structure (28) of any one of claims 9 to 11, further comprising a biasing element for biasing the first pad (78) and/or the second pad (80) towards the cylindrical shaft element (72).

13. The angulation structure (28) of any one of claims 9 to 12, wherein the first pad (78) and/or the second pad (80) are arranged to face towards an outer surface of the cylindrical shaft element (72).

14. The angulation structure (28) of claim 13, wherein an annular friction element (82) is arranged on the cylindrical shaft element (72), and the first pad (78) and/or the second pad (80) engage the annular friction element (82) to prevent rotation of the angulation structure (28), wherein the annular friction element (82) is in particular an elastic O-ring pulled onto the cylindrical shaft element (72).

15. An assembly method for an endoscope (10) comprising:
providing a body (26) adapted to be held by a human operator and to be coupled to an insertion tube (14) of the endoscope (10), wherein an angulation assembly (24) with an angulation structure (28) is rotatably mounted inside the body (26),
supporting a first control wire (30) and a second control wire (32) on the angulation structure (28), wherein the first control wire (30) and the second control wire (32) extend from opposite sides of the angulation structure (28) through the body (26) to the insertion tube (14), such that rotation of the angulation structure (28) winds one of the first control wire (30) and the second control wire (32) and unwinds the other one of the first control wire (30) and the second control wire (32);
connecting first ends of the first control wire (30) and the second control wire (32) to a distal end (22) of the insertion tube (14),
connecting second ends of the first control wire (30) and the second control wire (32) to the angulation structure (28), and
tensioning the first control wire (30) and the second control wire (32) using a tensioning mechanism mounted in the body (26) and coupled to the first control wire (30) and the second control wire (32) through a motion of the tensioning mechanism.
